# EUROPEAN PATENT SPECIFICATION

(11) **EP 1 706 099 B1**
(45) Date of publication and mention of the grant of the patent: **04.05.2011**
(21) Application number: 04801707.3
(22) Date of filing: 02.12.2004
(51) Int. Cl.: A61K 9/14, A61M 15/00, A61J 1/00, A61K 31/46, A61P 11/06, A61P 11/08

(54) **A MEDICAL PRODUCT COMPRISING TIOTROPIUM IN A MOISTURE-PROOF CONTAINER**
MEDIZINISCHES PRODUKT MIT TIOTROPIUM IN EINEM FEUCHTIGKEITSDICHTEN BEHÄLTER
PRODUIT MEDICAL CONTENANT DU TIOTROPIUM DANS UN CONTENANT ETANCHE A L'HUMIDITE

(30) Priority: 03.12.2003 SE 0303269
(43) Date of publication of application: 04.10.2006
(73) Proprietor: Boehringer Ingelheim International GmbH, 55216 Ingelheim am Rhein (DE)
(72) Inventor: NILSSON, Thomas, S-647 32 Mariefred (SE); MYRMAN, Mattias, S-13542 Tyresö (SE); NIEMI, Alf, S-645 50 Strängnäs (SE); CALANDER, Sven, 645-32 Strängnäs (SE)
(74) Representative: Eckhardt, Conrad
(86) International application number: PCT/SE2004/001790
(87) International publication number: WO 2005/053644

(56) References cited:
- WO-A1-01/27210
- WO-A1-03/084502
- WO-A2-00/74754
- US-A1- 2003 136 405

## Description

### TECHNICAL FIELD

The present invention relates to a medical product comprising inhalable doses of tiotropium loaded in a moisture-tight, dry container and in particular, a metered dry powder medicinal dose of tiotropium bromide being adapted for administration by a dry powder inhaler device.

### BACKGROUND

Asthma and chronic obstructive pulmonary disease (COPD) affect more than 30 million people in the United States. More than 100,000 deaths each year are attributable to these conditions. Obstruction to airflow through the lungs is the characteristic feature in each of these airway diseases, and the medications utilized in treatment are often similar.

Chronic obstructive pulmonary disease (COPD) is a widespread chronic lung disorder encompassing chronic bronchitis and emphysema. The causes of COPD are not fully understood. Experience shows that the most important cause of chronic bronchitis and emphysema is cigarette smoking. Air pollution and occupational exposures may also play a role, especially when combined with cigarette smoking. Heredity also causes some emphysema cases, due to alpha1 anti-trypsin deficiency.

Administration of asthma drugs by an oral inhalation route is very much in focus today, because of advantages offered like rapid and predictable onset of action, cost effectiveness and high level of comfort for the user. Dry powder inhalers (DPI) are especially interesting as an administration tool, compared to other inhalers, because of the flexibility they offer in terms of nominal dose range, i.e. the amount of active substance that can be administered in a single inhalation.

Anticholinergic agents, e.g. tiotropium, especially tiotropium bromide, are effective bronchodilators. These medicaments have a relatively fast onset and long duration of action, especially tiotropium bromide, which may be active for up to 24 hours. Anticholinergic agents reduce vagal cholinergic tone of the smooth muscle, which is the main reversible component of COPD. Anticholinergic agents have been shown to cause quite insignificant side effects in clinical testing, dryness of mouth and constipation are perhaps the most common symptoms. Because it is often very difficult to diagnose asthma and COPD correctly and since both disorders may co-exist, it is advantageous to treat patients suffering temporary or continuous bronchial obstruction resulting in dyspnoea with a small but efficient dose of a long-acting anticholinergic agent, preferably tiotropium bromide, because of the small adverse side effects.

Tiotropium bromide is the preferred anticholinergic agent because of its high potency and long duration. However, tiotropium is difficult to formulate in dry powder form to provide acceptable performance in terms of dose efficacy using prior art DPIs. Dose efficacy depends to a great deal on delivering a stable and high fine particle dose (FPD) out of the dry powder inhaler. The FPD is the respirable dose mass out of the dry powder inhaler with an aerodynamic particle size below 5 µm. Thus, when inhaling a dose of dry medication powder it is important to obtain by mass a high fine particle fraction (FPF) of particles with an aerodynamic size preferably less than 5 µm in the inspiration air. The majority of larger particles (>5 µm) does not follow the stream of air into the many bifurcations of the airways, but get stuck in the throat and upper airways, where the medicament is not giving its intended effect, but may instead be harmful to the user. It is also important to keep the dosage to the user as exact as possible and to maintain a stable efficacy over time, and that the medicament dose does not deteriorate during normal storage. For instance, Boehringer Ingelheim KG (BI) markets tiotropium bromide under the proprietary name of Spiriva^{®}. Surprisingly, in a recent investigation into the inhalability of Spiriva^{®} we have found that the Spiriva^{®}/HandiHaler^{®} system from BI for administration by inhalation of doses contained in gelatin capsules shows poor performance and has short in-use stability.

Thus, there is a need for improvement regarding a medical product comprising inhalable dry powder doses of tiotropium bromide, for instance Spirivao, and suitably adapted inhaler devices for the purpose of administration.

### SUMMERY

The present invention discloses a medical product for use in the treatment of respiratory disorders, and comprises a metered dose of a tiotropium, dry powder formulation, directly loaded and sealed into a moisture-tight, dry container acting as a dry, high barrier seal against moisture, the high barrier seal comprising aluminium. The container itself does not emit water, which may affect the tiotropium powder inside.

Thus, the container does not release any water to the dose and ingress of moisture from the exterior into the container is thereby prevented.

The dose of tiotropium is further intended for inhalation and the container is so dry and tight that the efficacy of the dose when delivered is unaffected by moisture.

In another aspect of the invention a type of inhaler is disclosed, which may accept at least one sealed, moisture-tight, dry container of a dose of tiotropium, e. g. Spiriva^{®}, and deliver said dose with a consistent FPD, over the expected shelf life of the product.

In a further aspect of the invention tiotropium may be mixed or formulated with at least one additional pharmacologically active ingredient (s) with an object of combining tiotropium with other medicament (s) to be used in the treatment of respiratory disorders. The present invention encompasses such use of tiotropium, in a combination of medicaments directly loaded into a sealed, moisture-tight, dry container for insertion into a DPI, the combination adapted for inhalation by the user.

The present medical product is set forth by the independent claims 1 and 2 and the dependent claims 3 to 11, and a pharmaceutical combination is set forth by the independent claims 12 and 13 and the dependent claims 14 to 22.

### BRIEF DESCRIPTION OF THE DRAWINGS

The invention, together with further objects and advantages thereof, may best be understood by referring to the following detailed description taken together with the accompanying drawings, in which:
- FIG. 1: illustrates in a graph the results of tests S1 to S5 and HB-S1 to HBS3;
- FIG. 2: illustrates in top and side views a first embodiment of a dose deposited onto a dose bed and a high barrier seal; and

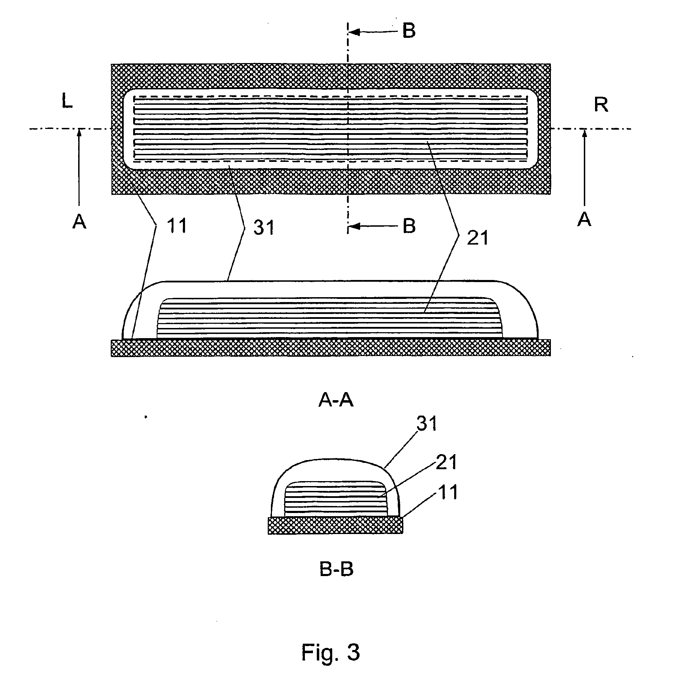

- FIG. 3: illustrates in top and side views a second embodiment of a dose onto a dose bed and a high barrier seal.

### DETAILED DESCRIPTION

Tiotropium is a new important anticholinergic substance for treatment of asthma and COPD but tiotropium is known in the industry to have problems maintaining in-use stability due to sensitivity to moisture. This fact is also documented in the report 'COLLEGE TER BEOORDELING VAN GENEESMIDDELEN MEDICINES EVALUATION BOARD; PUBLIC ASSESSMENT REPORT; Spiriva 18 µg, inhalation powder in hard capsules; RVG 26191' (2002-05-21) on page 6/28 under 'Product development and finished product' a very short in-use stability of the Spiriva^{®} product (9 days) is reported and a brittleness of the capsule in the blister pack and a very low FPD: 'about 3 ug'.

Details about an inhalation kit comprising inhalable powder of tiotropium and use of an inhaler for the administration of tiotropium may also be studied in the international publication WO 03/084502 A1. Details about tiotropium compounds, medicaments based on such compounds, the use of compounds and processes for preparing compounds may be studied in the European Patent Application 0 418 716 B1.

In the light of the above information given in the quoted report a test program was set up for the physical stability of the Spiriva^{®} product with respect to the compatibility of the formulation together with the components of the device according to Food and Drug Administration (FDA) 'Guidance for Industry; Metered Dose Inhaler (MDI) and Dry Powder Inhaler (DPI) Drug Products; Chemistry, Manufacturing, and Controls Documentation' page 37/62 'Drug product stability' lines 1209 - 1355. In 'Guidance for Industry; Stability Testing of Drug Substances and Drug Products; DRAFT GUIDANCE; B. Container/Closure' pages 35 and 36/110 lines 1127-1187, FDA states: 'Stability data should be developed for the drug product in each type of immediate container and closure proposed for marketing, promotion, or bulk storage. The possibility of interaction between the drug and the container and closure and the potential introduction of extractables into the drug product formulations during storage should be assessed during container/ closure qualification studies using sensitive and quantitative procedures.' and further 'Loss of the active drug substance or critical excipients of the drug product by interaction with the container/closure components or components of the drug delivery device is generally evaluated as part of the stability protocol. This is usually accomplished by assaying those critical drug product components, as well as monitoring various critical parameters (e.g., pH, preservative, effectiveness). Excessive loss of a component or change in a parameter will result in the failure of the drug product to meet applicable specifications.'

According to FDA publication 'Guidance for Industry; Stability Testing of Drug Substances and Drug Products' a 3 week test program in accelerated conditions (40 ± 2 °/ 75 ± 5 RH) for the container closure of the Spiriva^{®} product in this case the capsule and the blister pack and the impact of the capsule and the blister package on the FPD was set up and tested.

### Execution of tests

Spiriva^{®} powder formulation in bulk and Spiriva^{®} capsules from our local pharmacy where introduced to the laboratory together with the HandiHaler^{®}. The laboratory was set up to perform in-vitro tests according to European Pharmacopoeia (EP) and US Pharmacopoeia (USP) using two Andersen cascade impactors. All analytical work where then performed according to standardized methods for Physical Tests and Determinations for Aerosols, metered-dose inhalers and dry powder inhalers described in pharmacopoeias (e.g. USP 2002 <601>) using a state of the art High Performance Liquid Chromatograph (HPLC) system.

### Spiriva^{®} tests

### Test S1

Aerodynamic fine particle fraction of metered and delivered dose out of Handihaler^{®} using Spiriva^{®} formulation from bulk powder loaded into originator capsules during relative humidity below 10 %. The test was performed with 4 kPa pressure drop over the HandiHaler^{®} at room temperature and laboratory ambient conditions.

### Test S2

Aerodynamic fine particle fraction of metered and delivered dose out of Handihaler^{®} using commercial Spiriva^{®} capsules purchased from our local pharmacy. Test performed with 4 kPa pressure drop over the HandiHaler^{®} at room temperature and laboratory ambient conditions.

### Test S3

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 4 days into 40°C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### Test S4

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 13 days into 40°C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### Test S5

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 21 days into 40°C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### High barrier seal tests

### Test HBS1

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The aluminum containers were put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### Test HBS2

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The sealed aluminum containers were put into climate chambers for 7 days at 40 °C and 75 % Rh. The aluminum containers were put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### Test HBS3

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The sealed aluminum containers were put into climate chambers for 14 days at 40 °C and 75 % Rh. The aluminum containers were then put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### C-haler DPI.tests

A test was also made outside the stability test program to evaluate our proprietary inhaler, the so-called C-haler, in comparison with the HandiHaler^{®} using a tiotropium formulation. The C-haler cartridge used high barrier seals made out of aluminum foils from Alcan Singen Germany and the containers where filled volumetrically with 5 mg of the Spiriva^{®} powder formulation in bulk. The test was performed using a 4 kPa pressure drop over the C-haler at room temperature and laboratory ambient conditions. The results from the Andersen impactor tests were calculated on fine particle fraction based on delivered dose as well as on metered dose and converted to FPD. The results are given in Table 1 below.

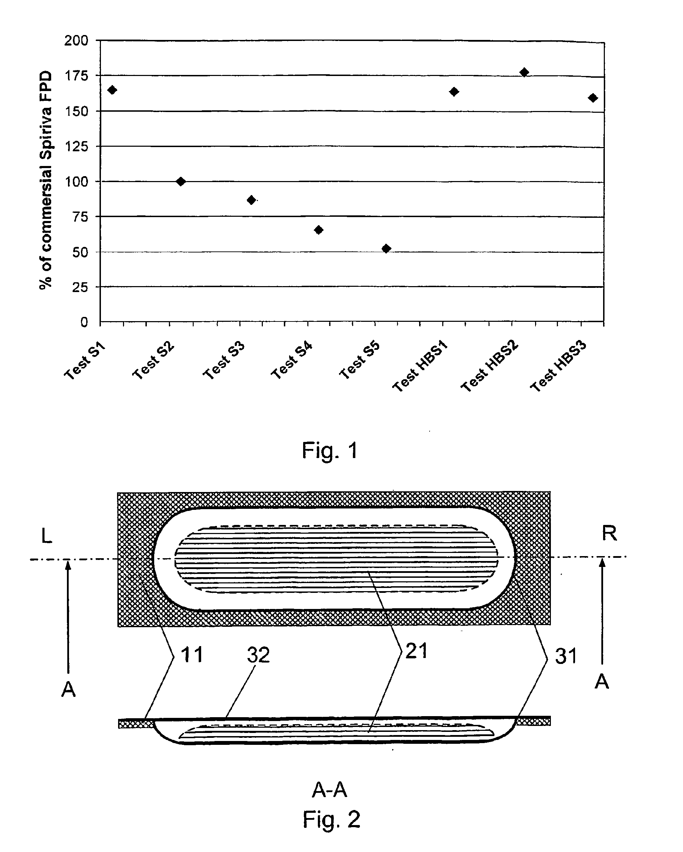

The results of tests S1-5 and HBS 1-3 are plotted in Figure 1. The Y-axis is designated '% of commercial Spiriva^{®} FPD'. This relates to the FPD out from the Handihaler^{®}, where 100 % is the FPD from a fresh sample from the pharmacy.

**Table 1. Inhaled fine particle dose (FPD) <5 µm in %**

| Calculation based on | Spiriva^{®} in HandiHaler^{®}, commercial sample, FPD | Spiriva^{®} in C-haler, FPD |
|---|---|---|
| Metered dose | 18% | 47% |
| Delivered dose | 36 % | 56 % |

| | | |
|---|---|---|
| Conclusion of the tests performed on Spiriva^{®} | | |

Surprisingly we have found and concluded in our tests that tiotropium is extremely sensitive to moisture and that a conventional packaging into gelatin capsules used for a majority of respiratory products will seriously affect the FPD. The results show that there is a need for a dry, moisture-tight high barrier seal enclosing the tiotropium formulation to preserve the original fine particle fraction. Not so surprisingly in the light of these findings, we have also found that the tiotropium formulation must be properly protected also during the in-use time if further reduction of the FPD shall be avoided. Eliminating the gelatin capsule has an unexpected, big, positive effect on the performance of the Spiriva^{®} formulation.

The tests carried out show that the moisture content of the gelatin capsule reduces the FPD out of the HandiHaler^{®} with approximately 50 % from the time of loading the dose into a capsule until the point in time when the product reaches the market. Loading Spiriva^{®} doses into dry containers made of materials presenting high barrier seal properties and then storing the loaded containers in 40°C and 75 % Rh, before transferring the Spiriva^{®} doses to originator capsules and performing the same tests using HandiHaler^{®} as before, no change can be detected in the fine particle dose (FPD), even after long periods of time. The FPD of Spiriva^{®} in gelatin capsules, however, is further diminishing during the in-use time of the product and the FPD has been shown to drop up to another 20 % after 5 days of storage in 40°C and 75 % Rh in an in-use stability test, due to the breaking of the moisture barrier in the opened blister secondary package. Table 1 shows that our propertiary C-haler using high barrier containers shows a 2.6 times higher performance than HandiHaler^{®} with respect to FPD based on metered dose.

### State of the art

Metered doses of the Spiriva^{®} powder formulation are today at the originator manufacturing site loaded into gelatin capsules. A gelatin capsule contains typically 13-14 % water by weight in the dose forming stage and after the capsules have been loaded they are dried in a special process in order to minimize water content. A number of dried capsules are then put in a common blister package. Details about suitable state-of-the-art capsule materials and manufacturing processes may be studied in the German Patent Application DE 101 26 924 A1. The remaining small quantity of water in the capsule material after drying is thus enclosed in the blister package and some water will be released into the enclosed air, raising the relative humidity in the air. The equilibrium between the captured air inside the package and the gelatin capsule will generate a relative humidity inside the blister package that will negatively affect the FPD of tiotropium powder out of the dry powder inhaler.

It is interesting to note that the big majority of dry powder formulations of many kinds of medicaments are not seriously affected by enclosed moisture in the capsule material or by normal storage variations in the relative humidity of the surrounding air. Surprisingly, our investigation has shown tiotropium to be very much different. Tiotropium powder is very much affected by very small amounts of water such that it tends to stick to wall surfaces and to agglomerate. By some mechanisms the FPD becomes less over time. Since the capsules are only used as convenient, mechanical carriers of Spiriva^{®} doses, a solution to the moisture problem would be not to use capsules at all, but rather to directly load doses into containers made of dry packaging material with high barrier seal properties during dry ambient conditions, preferably below 10% Rh.

The present invention discloses a dry, moisture-tight, directly loaded and sealed container enclosing a metered dose of tiotropium powder or a pharmaceutically acceptable salt, enantiomer, racemate, hydrate, or solvate, including mixtures thereof, and particularly tiotropium bromide, optionally further including excipients. The term"tiotropium"is in this document a generic term for all active forms thereof, including pharmaceutically acceptable salts, enantiomers, racemates, hydrates, solvates or mixtures thereof and may further include excipients for whatever purpose. The container uses dry, high barrier seals impervious to moisture and other foreign matters and is adapted for insertion into a dry powder inhaler device or the container may be adapted to be a part of an inhaler device.

"Dry"means that the walls of the container are constructed from selected materials such that the walls, especially the inside wall of the container, cannot release water that may affect the tiotropium powder in the dose such that the FPD is reduced. As a logical consequence container construction and materials should not be selected among those suggested in the German publication DE 101 26 924 A 1.

"High barrier seal"means a dry packaging construction or material or combinations of materials, comprising aluminium. A high barrier seal is characterized in that it represents a high barrier against moisture and that the seal itself is'dry', i. e. it cannot give off measurable amounts of water to the load of powder.

A "high barrier container" is a mechanical construction made to harbour and enclose a dose of e.g. tiotropium. The high barrier container is built using high barrier seals constituting the walls of the container.

"Directly loaded" means that the metered dose of tiotropium is loaded directly into the high barrier container, i.e. without first loading the dose into e.g. a gelatin capsule, and then enclosing one or more of the primary containers (capsules) in a secondary package made of a high barrier seal material.

The high barrier containers to be loaded with tiotropium should preferably be made out of aluminum foils approved to be in direct contact with pharmaceutical products. Aluminum foils that work properly in these aspects generally consist of technical polymers laminated with aluminum foil to give the foil the correct mechanical properties to avoid cracking of the aluminum during forming. Sealing of the formed containers is normally done by using a thinner cover foil of pure aluminum or laminated aluminum and polymer. The container and cover foils are then sealed together using at least one of several possible methods, for instance:
using a heat sealing lacquer, through pressure and heat;
using heat and pressure to fuse the materials together;
ultrasonic welding of the materials in contact.

Tiotropium in pure form is a very potent drug and it is therefore normally diluted before dose forming by mixing with physiologically acceptable excipients, e.g. lactose, in selected ratio(s) in order to fit a preferred method of dose forming or loading. Details about inhalation powders containing tiotropium in mixtures with excipients, methods of powder manufacture, use of powder and capsules for powder may be studied in the international publication WO 02/30389 A1.

In a further aspect of the invention tiotropium may be mixed or formulated with one or more other pharmacologically active ingredient(s) with an object of combining tiotropium with other medicament(s) to be used in a treatment of respiratory disorders. The present invention encompasses such use of tiotropium when a combination of tiotropium and other medicaments are deposited and sealed into a dry, moisture-tight high barrier container intended for insertion into a DPI for inhalation by the user. Examples of interesting combinations of substances together with tiotropium could be inhalable steroids, nicotinamide derivatives, beta-agonists, beta-mimetics, anti-histamines, adenosine A2A receptors, PDE4 inhibitors, dopamine D2 receptor agonists.

The sealed, dry, high barrier container of the invention that is directly loaded with a formulation of tiotropium may be in the form of a blister and it may e.g. comprise a flat dose bed or a formed cavity in aluminum foil or a molded cavity in a polymer material, using a high barrier seal foil against ingress of moisture, e.g. of aluminum or a combination of aluminum and polymer materials. The sealed, dry, high barrier container may form a part of an inhaler device or it may be a separate item intended for insertion into an inhaler device for administration of doses.

An inhaler providing a prolonged delivery of a dose during the course of a single inhalation constitutes a preferred embodiment of an inhaler for the delivery of the tiotropium powder formulation, e.g. Spiriva^{®}. An Air-razor method as described in our publication US 2003/0192539 A1 is preferably applied in the inhaler to efficiently and gradually aerosolize the dose when delivered to the user. Surprisingly enough, applying an inhaler for a prolonged delivery and using the Air-razor method on a dose comprising tiotropium in Spiriva^{®} formulation results in a FPD at least twice as big as that from the state-of-the-art HandiHaler^{®}.

## Claims

1. A medical product comprising a dry powder dose of tiotropium directly loaded into a container, **characterized in that**
a dry, high barrier seal constitutes the container,
the high barrier seal of the container, comprising aluminium, prevents ingress of moisture whereby the original fine particle fraction of the tiotropium powder dose is preserved, and
the dry powder dose in the container is adapted for administration by a dry powder inhaler.

2. A medical product comprising tiotropium and separate or together with at least one additional active pharmaceutical ingredient and optionally including excipients in a dry powder medical combination dose directly loaded into a container, **characterized in that**
a dry, high barrier seal constitutes the container;
the high barrier seal of the container, comprising aluminium, prevents ingress of moisture whereby the original fine particle fraction of the combination dose is preserved;
the combination dose is adapted for administration by a dry powder inhaler, and
the at least one additional active pharmaceutical ingredient is selected from the following groups of substances: inhalable steroids, nicotinamide derivatives, beta-agonists, beta-mimetics, anti-histamines, adenosine A2A receptors, PDE4 inhibitors, dopamine D2 receptor agonists.

3. The medical product according to claims 1 or 2, **characterized in that** administration of the dry powder dose is performed by inhalation from a dry powder inhaler providing a prolonged dose delivery.

4. The medical product according to claims 1 or 2, **characterized in that** the tiotropium substance consists of one or more physiologically acceptable salts of tiotropium.

5. The medical product according to claims 1 or 2, **characterized in that** an included excipient is lactose.

6. The medical product according to claims 1 or 2, **characterized in that** formed or flat aluminum foils, optionally laminated with polymers, constitute the dry, high barrier seal.

7. The medical product according to claims 1 or 2, **characterized in that** a cavity molded from a polymer material together with a high barrier seal constitute the container providing it with high barrier seal properties.

8. The medical product according to claims 1 or 2, **characterized in that** the container is a part of a dry powder inhaler.

9. The medical product according to claims 1 or 2, **characterized in that** the container is a separate part adapted for insertion into a dry powder inhaler

10. The medical product according to claims 1 or 2, **characterized in that** the container is a separate part comprising a primary part adapted for insertion into a dry powder inhaler and a secondary part enclosing the primary part in a moisture-tight package.

11. A pharmaceutical composition comprising tiotropium or a physiologically acceptable salt thereof and a physiologically acceptable excipient, **characterized in that**
the composition is directly loaded and sealed into a dry, moisture-tight package or dry, high barrier container comprising aluminium in order to preserve the original fine particle fraction of the composition.

12. A pharmaceutical composition comprising tiotropium or a physiologically acceptable salt thereof and separate or together with at least one active pharmaceutical ingredient, optionally including physiologically acceptable excipients in a combination dry powder dose, **characterized in that**
the combination dry powder dose is directly loaded and sealed into a dry, moisture tight package or dry, high barrier container comprising aluminium, in order to preserve the original fine particle fraction (FPF) of the composition;
the at least one active pharmaceutical ingredient is selected from the following groups of substances: inhalable steroids, nicotinamide derivatives, beta antagonists, beta-mimetics, anti-histamines, adenosine A2A receptors, PDE4 inhibitors, dopamine D2 receptor agonists.

13. The pharmaceutical composition according to claim 12, **characterized in that** administration of the combination dry powder dose is performed by inhalation from a dry powder inhaler providing a prolonged dose delivery.

14. The pharmaceutical composition according to claim 12 or 13, **characterized in that** the tiotropium comprises one or more physiologically acceptable salts of tiotropium.

15. The pharmaceutical composition according to claim 12 or 13, **characterized in that** an included excipient is lactose.

16. The pharmaceutical composition according to claim 12 or 13, **characterized in that** formed or flat aluminum foils, optionally laminated with polymers, constitute the dry, moisture-tight package or dry, high barrier container.

17. A pharmaceutical composition according to claim 12 or 13, **characterized in that** a cavity molded from a polymer material together with a high barrier seal constitute the dry, moisture-tight package or dry, high barrier container, thereby giving the package or container high barrier seal properties.

18. The pharmaceutical composition according to claim 12 or 13, **characterized in that** the dry, moisture-tight package or dry, high barrier container constitutes a part of a dry powder inhaler.

19. The pharmaceutical composition according to claim 12 or 13, **characterized in that** the dry, moisture-tight package or dry, high barrier container is a separate part adapted for insertion into a dry powder inhaler.

20. The pharmaceutical composition according to claim 12 or 13, **characterized in that** the dry, moisture-tight package or dry, high barrier container is a separate part comprising a primary package adapted for insertion into a dry powder inhaler, and a secondary moisture-tight package or container enclosing the primary package.

## Patentansprüche

1. Medizinisches Produkt, umfassend eine Trockenpulverdosis Tiotropium, die direkt in einen Behälter gefüllt ist, **dadurch gekennzeichnet, dass**
eine trockene hochwirksame Abdichtung des Behälters vorliegt;
die hochwirksame Abdichtung des Behälters, umfassend Aluminium, das Eindringen von Feuchtigkeit verhindert, wodurch die ursprüngliche Fraktion feiner Partikel der Tiotropiumpulverdosis geschützt bzw. erhalten wird, und
die Trockenpulverdosis im Behälter zur Verabreichung durch einen Trockenpulverinhalator angepasst ist.

2. Medizinisches Produkt, umfassend Tiotropium und getrennt davon oder zusammen mit mindestens einem zusätzlichen pharmazeutischen Wirkstoff und gegebenenfalls einschließlich Hilfsstoffen in einer medizinischen Trockenpulverkombination, die direkt in einen Behälter gefüllt ist, **dadurch gekennzeichnet, dass**
eine trockene hochwirksame Abdichtung des Behälters vorliegt;
die hochwirksame Abdichtung des Behälters, umfassend Aluminium, das Eindringen von Feuchtigkeit verhindert, wodurch die ursprüngliche Fraktion feiner Partikel der Kombinationsdosis geschützt bzw. erhalten wird;
die Kombinationsdosis zur Verabreichung durch einen Trockenpulverinhalator angepasst ist, und
mindestens einer der zusätzlichen pharmazeutischen Wirkstoffe ausgewählt ist aus der folgenden Gruppe von Substanzen: inhalierbaren Steroiden, Nikotinamidderivaten, beta-Agonisten, beta-Mimetika, Antihistaminika, Adenosin-A2A-Rezeporen, PDE4-Inhibitoren, Dopamin-D2-Rezeptoragonisten.

3. Medizinisches Produkt nach Anspruch 1 oder 2, **dadurch gekennzeichnet, dass** die Verabreichung der Trockenpulverdosis durch Inhalation aus einem Trockenpulverinhalator, der eine verlängerte Dosisfreigabe bereitstellt, erfolgt.

4. Medizinisches Produkt nach Anspruch 1 oder 2, **dadurch gekennzeichnet, dass** die Tiotropiumsubstanz aus ein oder mehreren physiologisch akzeptablen Tiotropiumsalzen besteht.

5. Medizinisches Produkt nach Anspruch 1 oder 2, **dadurch gekennzeichnet, dass** ein enthaltener Hilfsstoff Lactose darstellt.

6. Medizinisches Produkt nach Anspruch 1 oder 2, **dadurch gekennzeichnet, dass** geformte oder flache Aluminiumfolien, gegebenenfalls laminiert mit Polymeren, die trockene hochwirksame Abdichtung bilden.

7. Medizinisches Produkt nach Anspruch 1 oder 2, **dadurch gekennzeichnet, dass** ein aus einem Polymermaterial geformter Hohlraum zusammen mit einer hochwirksamen Abdichtung den Behälter bildet, was diesen mit hochwirksamen Dichtungseigenschaften versieht.

8. Medizinisches Produkt nach Anspruch 1 oder 2, **dadurch gekennzeichnet, dass** der Behälter einen Teil eines Trockenpulverinhalators darstellt.

9. Medizinisches Produkt nach Anspruch 1 oder 2, **dadurch** gekenntzeichnet, dass der Behälter ein separates Teil darstellt, welches angepasst ist, um in einen Trockenpulverinhalator eingesetzt zu werden.

10. Medizinisches Produkt nach Anspruch 1 oder 2, **dadurch gekennzeichnet, dass** der Behälter ein separates Teil darstellt, umfassend einen ersten Teil, angepasst zum Einsetzen in einen Trockenpulverinhalator, und einen zweiten Teil, der den ersten Teil in einer feuchtigkeitsdichten Einheit um- bzw. einschließt.

11. Pharmazeutische Zusammensetzung, umfassend Tiotropium oder ein physiologisch akzeptables Salz davon und einen physiologisch akzeptablen Hilfsstoff, **dadurch gekennzeichnet, dass**
die Zusammensetzung direkt in eine trockene feuchtigkeitsdichte Einheit oder einen trockenen hochdichten Behälter, umfassend Aluminium, gefüllt und verschlossen wird, um die ursprüngliche Fraktion feiner Partikel der Zusammensetzung zu schützen bzw. zu erhalten.

12. Pharmazeutische Zusammensetzung, umfassend Tiotropium oder ein physiologisch akzeptables Salz davon und getrennt davon oder zusammen mit mindestens einem pharmazeutischen Wirkstoff, gegebenenfalls enthaltend physiologisch akzeptable Hilfsstoffe, in einer Trockenpulverkombinationsdosis, **dadurch gekennzeichnet, dass** die Trockenpulverkombinationsdosis direkt in eine trockene feuchtigkeitsdichte Einheit oder einen trockenen hochdichten Behälter, umfassend Aluminium, gefüllt und verschlossen wird, um die ursprüngliche Fraktion feiner Partikel (FPF) der Zusammensetzung zu schützen bzw. zu erhalten;
wobei der mindestens eine pharrnazeutische Wirkstoff ausgewählt ist aus der folgenden Gruppe von Substanzen: inhalierbaren Steroiden, Nikotinamidderivaten, beta-Agonisten, beta-Mimetika, Antihistaminika, Adenosin-A2A-l2ezeporen, PDE4-Inhibitoren, Dopamm-D2-Rezeptoragonisten.

13. Phannazeutische Zusammensetzung nach Anspruch 12, **dadurch gekennzeichnet, dass** die Verabreichung der Trockenpulverkombinationsdosis durch Inhalation aus einem Trockenpulverinhalator, der eine verlängerte Dosisfreigabe bereitstellt, erfolgt.

14. Pharmazeutische Zusammensetzung nach Anspruch 12 oder 13, **dadurch gekennzeichnet, dass** das Tiotropium ein oder mehrere physiologisch akzeptable Tiotropiumsalze aufweist.

15. Pharmazeutische Zusammensetzung nach Anspruch 12 oder 13, **dadurch gekennzeichnet, dass** ein enthaltener Hilfsstoff Lactose darstellt.

16. Pharmazeutische Zusammensetzung nach Anspruch 12 oder 13, **dadurch gekennzeichnet, dass** geformte oder flache Aluminiumfolien, gegebenenfalls laminiert mit Polymeren, die trockene feuchtigkeitsdichte Einheit oder den trockenen hochdichten Behälter bilden.

17. Pharmazeutische Zusammensetzung nach Anspruch 12 oder 13, **dadurch gekennzeichnet, dass** ein aus einem Polymermaterial geformter Hohlraum zusammen mit einer hochwirksamen Abdichtung die trockene feuchtigkeitsdichte Einheit oder den trockenen hochdichten Behälter bildet, wodurch die Einheit oder der Behälter mit hochwirksamen Dichtungseigenschaften ausgestattet wird.

18. Pharmazeutische Zusammensetzung nach Anspruch 12 oder 13, **dadurch gekennzeichnet, dass** die trockene feuchtigkeitsdichte Einheit oder der trockene hochdichte Behälter einen Teil eines Trockenpulverinhalators darstellt.

19. Pharmazeutische Zusammensetzung nach Anspruch 12 oder 13, **dadurch gekennzeichnet, dass** die trockene feuchtigkeitsdichte Einheit oder der trockene hochdichte Behälter ein separates Teil darstellt, welches angepasst ist, um in einen Trockenpulverinhalator eingesetzt zu werden.

20. Pharmazeutische Zusammensetzung nach Anspruch 12 oder 13, **dadurch gekennzeichnet, dass** die trockene feuchtigkeitsdichte Einheit oder der trockene hochdichte Behälter ein separates Teil darstellt, umfassend eine erste Einheit, angepasst zum Einsetzen in einen Trockenpulverinhalator, und eine zweite feuchtigkeitsdichte Einheit oder einen Behälter, die/der die erste Einheit um- bzw einschließt.

## Revendications

1. Produit médical comprenant une dose de poudre sèche de tiotropium introduite directement dans un récipient, **caractérisé en ce que** un dispositif d'étanchéité formant forte barrière, sec, constitue le récipient;
le dispositif d'étanchéité formant forte barrière du récipient, comprenant de l'aluminium, empêche la pénétration d'humidité de sorte que la fraction de fines particules originelle de la poudre de tiotropium est préservée, et
la dose de poudre sèche dans le récipient est adaptée à l'administration par un inhalateur de poudre sèche.

2. Produit médical comprenant du tiotropium et séparé ou ensemble avec au moins un ingrédient pharmaceutique actif supplémentaire et incluant éventuellement des excipients dans une dose de combinaison médicale de poudre sèche introduite directement dans un récipient, **caractérisé en ce que** un dispositif d'étanchéité formant forte barrière, sec, constitue le récipient;
le dispositif d'étauchéité formant forte barrière du récipient, comprenant de l'aluminium, empêche la pénétration d'humidité de sorte que la fraction de fines particules originelle de dose de combinaison est préservée ;
la dose de combinaison est adaptée pour l'administration par un inhalateur de poudre sèche, et
le au moins un ingrédient pharmaceutique actif supplémentaire est choisi dans les groupes de substances suivants : stéroïdes inhalables, dérivé du nicotinamide, bêta-agonistes, bêta-mimétiques, anti-histaminiques, récepteurs d'adénosine A2A, inhibiteurs de PDE4, agonistes de récepteur de dopamine D2.

3. Produit médical selon la revendication 1 ou 2 **caractérisé en ce que** l'administration de la dose de poudre sèche est accomplie par inhalation depuis un inhalateur de poudre sèche assurant une dérivrance de dose prolongée.

4. Produit médical selon la revendication 1 ou 2 **caractérisé en ce que** la substance tiotropium consiste en un ou plusieurs sels de tiotropium physiologiquement acceptables.

5. Produit médical selon la revendication 1 ou 2 **caractérisé en ce qu'**un excipient inclus est le lactose.

6. Produit médical selon la revendication 1 ou 2 **caractérisé en ce que** des feuilles d'aluminium formées ou planes, éventuellement stratifiées avec des polymères, constituent le dispositif d'étanchéité formant forte barrière, sec.

7. Produit médical selon la revendication 1 ou 2 **caractérisé en ce qu'**une cavité moulée en une matière polymère en même temps qu'un dispositif d'étanchéité formant forte barrière constituent le récipient en lui conférant des propriétés de dispositif d'étanchéité formant forte barrière.

8. Produit médical selon la revendication 1 ou 2 **caractérisé en ce que** le récipient est une partie d'un inhalateur de poudre sèche.

9. Produit médical selon la revendication 1 ou 2 **caractérisé en ce que** le récipient est une partie séparée adaptée pour l'insertion dans un inhalateur de poudre sèche.

10. Produit médical selon la revendication 1 ou 2 **caractérisé en ce que** le récipient est une partie séparée comprenant une partie primaire adaptée pour l'insertion dans un inhalateur de poudre sèche et une partie secondaire entourant la partie primaire dans un emballage étanche à l'humidité.

11. Composition pharmaceutique comprenant du tiotropium ou un sel physiologiquement acceptable de celui-ci et un excipient physiologiquement acceptable, **caractérisée en ce que**
la composition est directement introduite et enfermée de manière étanche dans un emballage étanche à l'humidité, sec, ou un récipient formant forte barrière, sec, comprenant de l'aluminium pour préserver la fi-action de fines particules originelle de la composition.

12. Composition pharmaceutique comprenant du tiotropium ou un sel physiologiquement acceptable de celui-ci et séparé ou ensemble avec au moins un ingrédient pharmaceutique actif, incluant éventuellement des excipients physiologiquement acceptables dans une dose de poudre sèche de combinaison, **caractérisée en ce que**
la dose de poudre sèche de combinaison est directement introduite et enfermée de manière étanche dans un emballage étanche à l'humidité, sec, ou un récipient formant forte barrière, sec, comprenant de l'aluminium, pour préserver la fraction de fines particules (FFP) originelle de la composition ;
le au moins un ingrédient pharmaceutique actif est choisi dans les groupes de substances suivants : stéroïdes inhalables, dérivé du nicotinamide, bêta-agonistes, bêta-mimétiques, anti-histaminiques, récepteurs d'adénosine A2A, inhibiteurs de PDE4, agonistes de récepteur de dopamine D2.

13. Composition pharmaceutique selon la revendication 12 **caractérisée en ce que** l'administration de la dose de poudre sèche de combinaison est accomplie par inhalation depuis un inhalateur de poudre sèche assurant une dérivrance de dose prolongée.

14. Composition pharmaceutique selon la revendication 12 ou 13 **caractérisée en ce que** le tiotropium comprend un ou plusieurs sels de tiotropium physiologiquement acceptables.

15. Composition pharmaceutique selon la revendication 12 ou 13 **caractérisée en ce qu'**un excipient inclus est le lactose.

16. Composition pharmaceutique selon la revendication 12 ou 13 **caractérisée en ce que** des feuilles d'aluminium formées ou planes, éventuellement stratifiées avec des polymères, constituent l'emballage étanche à l'humidité, sec, ou le récipient formant forte barrière, sec.

17. Composition pharmaceutique selon la revendication 12 ou 13 **caractérisée en ce qu'**une cavité moulée en une matière polymère en même temps qu'un dispositif d'étanchéité formant forte barrière constituent l'emballage étanche à l'humidité, sec, ou le récipient formant forte barrière, sec, en conférant à l'emballage ou au récipient des propriétés de dispositif d'étanchéité formant forte barrière.

18. Composition pharmaceutique selon la revendication 12 ou 13 **caractérisée en ce que** l'emballage étanche à l'humidité, sec, ou le récipient formant forte barrière, sec, constitue une partie d'un inhalateur de poudre sèche.

19. Composition pharmaceutique selon la revendication 12 ou 13 **caractérisée en ce que** l'emballage étanche à l'humidité, sec, ou le récipient format forte barrière, sec, est une partie séparée adaptée pour l'insertion dans un inhalateur de poudre sèche.

20. Composition pharmaceutique selon la revendication 12 ou 13 **caractérisée en ce que** l'emballage étanche à l'humidité, sec, ou le récipient formant forte barrière, sec, est une partie séparée comprenant un emballage primaire adapté à l'insertion dans un inhalateur de poudre sèche et un emballage secondaire ou récipient étanche à l'humidité entourant l'emballage primaire.
